(19) Europäisches Patentamt
European Patent Office
Office européen des brevets

(11) **EP 2 065 075 A1**

(12) **EUROPEAN PATENT APPLICATION**

(43) Date of publication:
**03.06.2009 Bulletin 2009/23**

(21) Application number: **09000612.3**

(22) Date of filing: **18.01.2002**

(51) Int Cl.:
***A61Q 19/00*** *(2006.01)* ***A61K 8/04*** *(2006.01)*
***A61K 8/06*** *(2006.01)* ***A61K 8/26*** *(2006.01)*
***A61K 8/41*** *(2006.01)* ***A61K 8/65*** *(2006.01)*
***A61K 8/73*** *(2006.01)*

(84) Designated Contracting States:
**DE FR GB IT**

(62) Document number(s) of the earlier application(s) in accordance with Art. 76 EPC:
**02715820.3 / 1 475 070**

(71) Applicant: **Shiseido Company, Ltd.**
**Chuo-ku**
**Tokyo 104-8010 (JP)**

(72) Inventors:
- **Nakamura, Tadashi**
  **Yokohama-shi**
  **Kanagawa 224-8558 (JP)**
- **Takasu, Emiko**
  **Yokohama-shi**
  **Kanagawa 224-8558 (JP)**
- **Kaneda, Isamu**
  **Yokohama-shi**
  **Kanagawa 224-8558 (JP)**

(74) Representative: **Hollatz, Christian**
**ter Meer Steinmeister & Partner GbR**
**Patentanwälte**
**Mauerkircherstrasse 45**
**81679 München (DE)**

Remarks:
This application was filed on 16-01-2009 as a divisional application to the application mentioned under INID code 62.

(54) **Water-in-oil emulsion composition and cosmetic emulsion comprising the same**

(57) The present invention is a water-in-oil emulsified composition comprising (a) 0.1-20 wt% organophilic clay mineral, (b) 10-70 wt% oil component, (c) 0.01-10 wt% emulsifier having an HLB value of not more than 7, and (d) 0. 1-90 wt% microgel having an average particle size of 0.1-1,000 micrometers obtained by dissolving a hydrophilic compound having a gelation ability in water or an aqueous component, letting it cool down and solidify to form a gel, and pulverizing said gel, as well as an emulsified cosmetic that uses this composition.

The present invention provides a water-in-oil emulsified composition that exhibits good emulsified states, does not change over different temperatures and/or time, has superior stability, and gives a non-sticky, fresh, and good tactile sensation during use, as well as an emulsified cosmetic using this composition.

EP 2 065 075 A1

## Description

TECHNICAL FIELD

The present invention relates to an water-in-oil emulsified composition and an emulsified cosmetic using this composition. More specifically, the present invention relates to a water-in-oil emulsified composition that exhibits good emulsified states, does not change over different temperatures and/or time, has superior stability, and gives a non-sticky, fresh, and good tactile sensation during use, as well as an emulsified cosmetic using this composition.

BACKGROUND ART

In the past, water-in-oil type emulsified cosmetic compositions having high stability have been obtained by increasing the viscosity of the outer phase (i.e., oil phase) by blending in solid and semisolid oil components. Thus, an oily and sticky sensation during use resulted, leading to poor evaluation results as a cosmetic.
The object of the present invention is to solve this problem and provide a water-in-oil emulsified composition that has superior stability and gives a non-sticky, fresh, and good tactile sensation during use, as well as an emulsified cosmetic using this composition.

DISCLOSURE OF INVENTION

For the purpose of solving the aforementioned problem, the present invention provides a water-in-oil emulsified composition comprising (a) 0.1-20 wt% organophilic clay mineral, (b) 10-70 wt% oil component, (c) 0.01-10 wt% emulsifier having an HLB value of not more than 7, and (d) 0.1-90 wt% microgel having an average particle size of 0.1-1,000 micrometers obtained by dissolving a hydrophilic compound having a gelation ability in water or an aqueous component, letting it cool down and solidify to form a gel, and pulverizing said gel.
In the aforementioned (d) ingredient, the hydrophilic compound having gelation ability is preferably one, two, or more selected from a group consisting of agar, carrageenan, curdlan, gelatin, gellan gum, and alginic acid.
As for the average particle size of the microgel of (d) ingredient, 1-300 micrometers is preferable.
In addition, the present invention provides an emulsified cosmetic comprising the aforementioned water-in-oil emulsified composition.

BEST MODE FOR CARRYING OUT THE INVENTION

The present invention is described in detail below.
Selection of the organophilic clay mineral used for (a) ingredient is not limited in particular as long as it can be generally used in cosmetics. In the present invention, the cation modified clay mineral obtained by treating a water swelling clay mineral with a quaternary ammonium salt cationic surfactant is preferably used.
Preferable examples of the aforementioned water swelling clay mineral include a clay mineral such as phyllosilicate from the smectide group having a three-layer structure such as colloidal aluminum silicate hydrate generally represented by the following general formula (I).

$$(X,Y)_{2-3}(Si,Al)_4O_{10}(OH)_2Z_{1/3}\cdot nH_2O \qquad (I)$$

(In this formula, X denotes Al, Fe(III), Mn(III), or Cr(III); Y denotes Mg, Fe (II), Ni, Zn, Li, or Mn (II); Z denotes K, Na, 1/2Ca, or 1/2Mg.) Specific examples include natural or synthetic (i. e. , the OH group is substituted with fluorine in formula (I)) montmorillonites such as montmorillonite, saponite and hectorite (commercially available products include Veegum from Vanderbild Co., Inc., Kunipia from Kunimine Kogyo KK, Laponite from Laporte, etc.) and synthetic mica such as sodium silicic mica, sodium or lithium teniorite (commercially available products include Dimonite available from Topy Kogyo K.K.), which can be preferably used. One, two, or more water swelling clay minerals can be used.
The quaternary ammonium salt cationic surfactant for treating the aforementioned water swelling clay mineral is a compound represented by the following general formula (II).

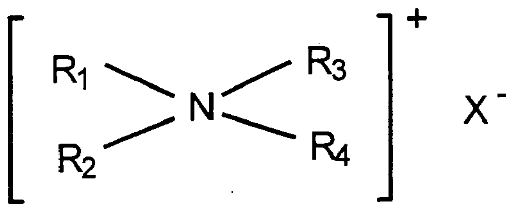

(II)

(In this formula, $R_1$ denotes an alkyl group having 10-20 carbon atoms or a benzyl group; $R_2$ denotes a methyl group or an alkyl group having 10-22 carbon atoms; $R_3$ and $R_4$ independently denote an alkyl group or hydroxyalkyl group having 1-3 carbon atoms; and X denotes a halogen atom or a methylsulfate residue.)

Specific examples include dodecyl trimethyl ammonium chloride, myristyl trimethyl ammonium chloride, cetyl trimethyl ammonium chloride, stearyltrimethyl ammonium chloride, alkyl trimethyl ammonium chloride, behenyl trimethyl ammonium chloride, myristyl dimethyl ethyl ammonium chloride, cetyl dimethyl ethyl ammonium chloride, stearyl dimethyl ethyl ammonium chloride, alkyl dimethyl ethyl ammonium chloride, behenyl dimethyl ethyl ammonium chloride, myristyl diethyl methyl ammonium chloride, cetyl diethyl methyl ammonium chloride, stearyl diethyl methyl ammonium chloride, alkyl diethyl methyl ammonium chloride, behenyl diethyl methyl ammonium chloride, benzyl dimethyl myristyl ammonium chloride, benzyl dimethyl cetyl ammonium chloride, benzyl dimethyl stearyl ammonium chloride, benzyl dimethyl behenyl ammonium chloride, benzyl methyl ethyl cetyl ammonium chloride, benzyl methyl ethyl stearyl ammonium chloride, distearyl dimethyl ammonium chloride, and dibehenyl dihydroxyethyl ammonium chloride, as well as the aforementioned compounds with their chloride replaced by bromide, and dipalmityl propyl ethyl ammonium sulfate. One, two, or more of these quaternary ammonium salt cationic surfactants can be selected at will for use.

As for a method of treating the aforementioned water swelling clay mineral with the quaternary ammonium salt cationic surfactant to obtain the cation modified clay mineral, for example, the aforementioned water swelling clay mineral and the quaternary ammonium salt cationic surfactant are dispersed and stirred in a low-boiling-point solvent such as water, acetone, or a lower alcohol and then the low-boiling-point solvent is removed. It is also possible to blend the water swelling clay mineral and the quaternary ammonium salt cationic surfactant and let organic modification occur in the recipe; such a product can also be used preferably. It is possible to use both a quaternary ammonium salt cationic surfactant and a nonionic surfactant.

A preferable amount of the quaternary ammonium salt cationic surfactant is 60-140 meq for 100 g of the water swelling clay mineral. When a nonionic surfactant is used, the amount to be used is preferably 5-100 g, more preferably 15-50 g, for 100 g of the water swelling clay mineral.

Representative examples of the cationic modified clay mineral obtained by treating the water swelling clay mineral with the quaternary ammonium salt cationic surfactant include dimethyl ammonium hectorite, benzyl dimethyl stearyl ammonium hectorite, and distearyl dimethyl ammonium chloride-treated aluminum magnesium silicate. Commercially available products include Benton 38 (montmorillonite modified with distearyldimethylammonium chloride, available from Nationalred Co.).

The blend ratio of (a) ingredient is preferably 0.1 to 20 wt%, more preferably 0.1-10 wt% of the total amount of the water-in-oil emulsified cosmetic. If it is less than 0.1 wt% then sufficient stability is hard to achieve. On the other hand, sufficient stability can be achieved with a blend ratio of 20 wt% or less; a higher blend ratio results in increased hardness that affects usability and reduces stability, which are not preferable. One, two or more of (a) ingredients can be used.

The oil component for (b) ingredient makes up the outer phase and selection thereof is not limited in particular among such components that are generally used in cosmetics; for example, liquid fats and oils, solid fats and oils, waxes, hydrocarbon oils, higher fatty acids, higher alcohols, synthesized ester oils, and silicone oils can be used at will.

Examples of the liquid fats and oils include avocado oil, tsubaki oil, turtle fatty acid, macadamia nut oil, corn oil, mink oil, olive oil, rape oil, egg yolk oil, sesame oil, persic oil, wheat germ oil, sasanqua oil, castor oil, linseed oil, safflower oil, cotton seed oil, perilla oil, soybean oil, peanut oil, tea seed oil, Japanese nutmeg oil, rice bran oil, Chinese wood oil, Japan wood oil, Jojoba oil, germ oil, triglycerin, glyceryl trioctanoate, and glycerol triisopalmitate.

Examples of the solid fats and oils include cacao butter, coconut oil, horse oil, hydrogenated coconut oil, palm oil, beef fat, sheep tallow, hydrogenated beef fat, palm kernel oil, pork tallow, beef bone tallow, Japanese core wax kernel oil, hydrogenated oil, neatsfoot oil, Japanese core wax, and hydrogenated castor oil.

Examples of the waxes include beeswax, candelilla wax, cotton wax, carnauba wax, bayberry wax, tree wax, whale wax, montan wax, bran wax, lanolin, kapok wax, lanolin acetate, liquid lanolin, sugar cane wax, lanolin fatty acid isopropyl ester, hexyl laurate, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether.

Examples of the hydrocarbon oils include liquid petrolatum, ozokerite, squalene, pristane, paraffin, ceresin,

squalene, petrolatum, and microcrystalline wax.

Examples of the higher fatty acids include lauric acid, myristic acid, palmitic acid, stearic acid, behenic acid, 12-hydroxy stearic acid, undecylenic acid, and tall oil.

Examples of higher alcohols include cetyl alcohol, stearyl alcohol, behenyl alcohol, myristyl alcohol, and cetostearyl alcohol.

Examples of the synthesized ester oils include isopropyl myristate, cetyl octanoate, octyldodecyl myristate, isopropyl palmitate, butyl stearate, hexyl laurate, myristyl myristate, decyl oleate, hexyl decyl dimethyloctanoate, cetyl lactate, myristyl lactate, lanolin acetate, isocetyl stearate, isocetyl isostearate, cholesteryl 12-hydroxystearate, ethylene glycol di-2-ethylhexylate, dipentaerythritol fatty acid ester, N-alkyl glycol monoisostearate, neopentyl glycol dicaprate, diisostearyl malate, glyceryl di-2-heptylundecanoate, trimethylolpropane tri-2-ethylhexylate, trimethylolpropane triisostearate, pentane erythritol tetra-2-ethylhexylate, glycerin tri-2-ethylhexylate, trimethylolpropane triisostearate, cetyl 2-ethyl hexanoate, 2- ethylhexyl palmitate, glyceryl trimyristate, tri-2- heptyl undecanoic acid glyceride, methyl castor oil fatty acid, oleic acid oil, cetostearyl alcohol, aceto glyceride, 2-heptylundecyl palmitate, diisobutyl adipate, N-lauroyl -L-glutamic acid -2-octyldodecyl ester, 2-heptylundexyl adipate, ethyl laurate, di-2-ethylhexyl cebacate, 2-hexyldecyl myristate, 2-hexyldecyl palmitate, 2-hexyldecyl adipate, diisopropyl cebacate, 2-ethylhexyl succinate, ethyl acetate, butyl acetate, amyl acetate, and triethyl citrate.

Examples of the silicone oils include chain silicone oils such as dimethylpolysiloxane, methylphenylpolysiloxane, and methyl hydrogen polysiloxane; and ringed silicone oils such as dodecamethyl cyclohexasiloxane, octamethyl cyclotetrasiloxane, decamethyl cyclopentasiloxane, and tetramethyl tetrahydrogen polysiloxane.

The blend ratio of (b) ingredient is preferably 10-70 wt% of the total amount of the water-in-oil emulsified cosmetic.

The emulsifier for (c) ingredient has a HLB of 7 or less. Those having a HLB higher than 7 have a high hydrophilicity and using these makes it hard to obtain a stable water-in-oil emulsified composition.

In the present invention, this HLB is calculated using the Kawakami method, which is represented by the following equation 1.

Equation 1

$$HLB = 7 + 11.7 \cdot \log(MW/MO)$$

(Where, MW denotes the molecular weight of the hydrophilic portion and MO denotes the molecular weight of the lipophilic portion.)

Examples of the emulsifier having a HLB of 7 or less include sorbitan fatty acid esters such as sorbitan monolaurate, sorbitan monoisostearate, sorbitan tristearate, which are surfactants; glycerin fatty acid esters such as glycerol monostearate and glycerol monooleate; polyoxyethylene hydrogenated castor oils such as POE (5) hydrogenated castor oil, POE (7.5) hydrogenated castor oil, and POE (10) hydrogenated castor oil; and polyether modified silicone surfactants, and dimethicone polyol.

The blend ratio of (c) ingredient is preferably 0.01-10 wt%, and more preferably 0.1-5 wt%, of the total amount of the emulsified composition of the present invention. If the blend ratio is too low then a stable emulsion cannot be obtained; on the other hand, if the blend ratio is too high then there are problems in terms of usability such as stickiness and lack of succulent sensation. One, two, or more of (c) ingredients can be selected at will for use.

This (c) ingredient may be contained in the composition of the present invention as being adsorbed on the organophilic clay mineral, i. e. (b) ingredient. That is, when preparing the water-in-oil emulsified composition or the emulsified cosmetic of the present invention, (c) ingredient may be blended in by treating the organophilic clay mineral with a surfactant having a HLB of 7 or less that is to be (c) ingredient so that an emulsifier having a HLB of 7 or less is adsorbed on the organophilic clay mineral.

In addition to the aforementioned essential ingredients, additives can be freely blended into the outer phase (oil phase) within the range that does not affect the effects of the present invention. For example, other oil components, oil-soluble polymers, powders, and polymer granules that are conventionally used in cosmetics, quasi drugs and such may be blended in.

The microgel for (d) ingredient with an average particle size of 0. 1-1, 000 micrometers, is obtained by dissolving a hydrophilic compound having a gelation ability in water or an aqueous component, letting it cool down and solidify to form a gel, and pulverizing said gel. Such (d) ingredient is contained in the inner phase (water phase).

In the aforementioned (d) ingredient, selection of the hydrophilic compound having a gelation ability is not limited in particular as long as it is a water soluble compound and can be used in the fields of cosmetics and medical drugs. Specific examples include hydrophilic proteins having a gelation ability such as gelatin and collagen, as well as

hydrophilic polysaccharides such as agar, curdlan, scleroglucan, schizophyllan, gellan gum, alginic acid, carrageenan, mannan, pectin, and hyaluronic acid. Of these, gelatin, agar, curdlan, gellan gum, alginic acid, and carrageenan are particularly preferable for use. One, two or more hydrophilic compounds having a gelation ability can be used.

These hydrophilic compounds having a gelation ability are then dissolved in water or an aqueous ingredient to form a gel. Dissolution of the aforementioned compound in water or an aqueous ingredient can be carried out by mixing, heating, etc. Gelation can be achieved by a conventional method, e. g. by discontinuing heating after dissolution and letting the temperature cool down to a point lower than the gelation temperature (solidification temperature).

Selection of the aqueous ingredient to be blended in is not limited in particular as long as it is an aqueous ingredient that can be used in the field of cosmetics and drugs; examples include glycols such as dipropylene glycol and propylene glycol, lower alcohols such as methanol, ethanol, and propanol, as well as ingredients that are generally blended in as water phase ingredients. Specific examples include, but not limited to, chelating agents such as metaphosphate and edetate, as well as pH adjusting agents and preservatives.

The gel strength of the aforementioned gel is not limited in particular as long as the gel itself can retain its shape or has enough strength for obtaining a microgel in the next process. In the present invention, the microgel can be obtained even with a fairly weak gel strength such as a jelly strength of 30 g/cm$^2$ (Nichikansui-shiki measuring method).

The gel formed as mentioned above is then crushed with a homogenizer, disper, mechanical stirrer, etc. to obtain the desired microgel. The average particle size of the microgel is 0.1-1,000 micrometers, preferably 1-300 micrometers, and more preferably 10-200 micrometers. The degree of crushing can be adjusted according to the purpose as long as the average particle size of the obtained microgel is not outside of the aforementioned range specified in the present invention; when smoother usability is required, thorough crushing is conducted by high speed stirring, and, on the other hand, when the tactile sensation of the microgel itself is required, the degree of crushing is reduced by lighter stirring to obtain a microgel with a relatively large particle size.

As for the viscosity of the microgel thus obtained, when agar is used as the hydrophilic compound having gelation ability for example, a range of 2, 000-1, 000, 000 mPa·s is preferable when a B-type viscometer (speed 0.6 rpm, 25˚C) is used for measurement with an agar concentration of approximately 0.5-2%. By setting the viscosity of the microgel in the aforementioned range, a superior sensation during use can be obtained in terms of smoothness and moistening sensation (water retention). If the viscosity is too low, then smoothness and the moistening sensation (water retention) tend to be poor.

The blend ratio of (d) ingredient is preferably 0.1-90 wt%, and more preferably 1-80 wt%, of the total amount of the emulsified composition of the present invention. If the blend ratio is too low, then succulent sensation during use cannot be obtained; on the other hand, if the blend ratio is too high, there are problems with usability due to, for example, a sticky sensation. One, two, or more of (d) ingredients can be selected at will for use.

When the water-in-oil emulsified composition of the present invention is used, for example, in an emulsified cosmetic, in addition to the aforementioned essential ingredients, other ingredients that are usually blended into cosmetics can be freely blended in as necessary within the range that does not affect the effects of the present invention. Such ingredients include humectants such as polyethylene glycol, glycerin, 1,3- butylene glycol, erythritol, sorbitol, xylitol, and maltitol; L-amino acids including L-alanine, β-alanine, L-arginine hydrochloride, L-aspartic acid monohydrate, L-sodium aspartate monohydrate, L-potassium aspartate dihydrate, L-aspartic acid, L-citrulline, L-glutamic acid, L-glutamic acid hydrochloride, L-sodium glutamate monohydrate, L-potassium glutamate monohydrate, L-glutamine, glycin, trimethyl glycin, L-histidine, L-histidine hydrochloride monohydrate, L-hydroxy proline, L-isoleucine, L-leucine, L-lysine, L-lysine hydrochloride, L-ornithine hydrochloride, L-proline, L-phenyl-alanine, L-serine, L-threonine, L-triptophan, L-tyrosine, L-valine, L-dopa, and L-α aminobutyric acid, as well as their hydrates; lower alcohols such as ethanol; antioxidants such as butylhydroxy toluene, tocopherol, and phytin antimicrobial agents/preservatives such as benzoic acid, salicylic acid, sorbic acid, paraoxybenzoic acid alkyl ester (ethylparaben, butylparaben, etc.) and hexachlorophene; Benzoic acid-type ultraviolet absorbents such as paraminobenzoic acid (PABA), PABA monoglycerin ester, N, N-dipropoxy PABA ethyl ester, N,N-diethoxy PABA ethyl ester, N,N-dimethyl PABA ethyl ester, and N,N-dimethyl PABA butyl ester; anthranilic acid-type ultraviolet absorbents such as homomentyl-N-acetyl anthranilate; salicylic acid-type ultraviolet absorbents such as amyl salicylate, menthyl salicylate, homomentyl salicylate, octyl salicylate, phenyl salicylate, benzil salicylate, and p-isopropanol phenyl salicylate; cinnamic acid-type ultraviolet absorbents such as octyl cinnamate, ethyl -4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate, 2-ethylhexyl-p-methoxy cinnamate, 2-ethoxyethyl-p-methoxy cinnamate, cyclohexyl-p-methoxy cinnamate, ethyl-α-cyano-β -phenyl cinnamate, 2-ethylhexyl-α-cyano--phenyl cinnamate, glyceryl mono-2-ethyl hexanoyl-diparamethoxy cinnamate, and 3-methyl-4-[methylbis (trimethyl siloxy) silyl] butyl 3,4,5- trimethoxy cinnamate; benzophenone-type ultraviolet absorbents such as 2,4-dihydroxybenzophenone, 2,2'-dihydroxy-4-methoxy benzophenone, 2,2'-dihydroxy-4,4'-dimethoxybenzophenone, 2,2', 4,4'-tetrahydroxy benzophenone, 2-hydroxy-4-methoxy benzophenone, 2-hydroxy-4-methoxy -4'-methylbenzophenone, 2-hydroxy -4-methoxy benzophenone-5-sulfonate, 4-phenyl benzophenone, 2-ethylhexyl-4'-phenyl-benzophenone-2-carboxylate, 2-hydroxy-4-n-octoxy benzophenone, and 4-hydroxy-3-carboxy benzophenone; ultraviolet light absorbents such as 3-(4'-methylbenzylidene)-d,1-camphor, 3-benzylidene-d,1-camphor, urocanic acid, urocanic acid ethyl ester, 2-phenyl-5-methyl benzoxazole, 2,2'-hydroxy-5-methylphenyl benzotriazol, 2-(2'-hydroxy-5'-t-octylphenyl) benzotriazol, 2-(2'-hydroxy-5'-methylphenyl benzotriazol, dibenzaladine, dianisoylmethane, 4-methoxy-4'-t-butyl dibenzoyl-methane, 5-(3,3-dimethyl-2-norbornylidene)-3-pentane-2-one, and terephthalylidene dicamphor sulfonic acid); organic acids such as acyl sarcosinic acid (sodium lauroyl sarcosinate, for example), glutathione, citric acid, malic acid, tartaric acid, and lactic acid; vitamins including vitamin A and its derivatives, vitamin B's such as vitamin $B_6$ hydrochloride, vitamin $B_6$ tripalmitate, vitamin $B_6$ dioctanoate, vitamin $B_2$ and its derivatives, vitamin $B_{12}$, and vitamin $B_{15}$ and its derivatives, vitamin C' s such as ascorbic acid, ascorbic acid sulfuric ester (salt), ascorbic acid phosphate (salt), and ascorbic acid dipalmitate, vitamin E' s such as $\alpha$-tocopherol, $\beta$-tocopherol, $\gamma$-tocopherol, and vitamin E acetate, vitamin D's, vitamin H, pantothenic acid, and pantethine; various drugs such as nicotinamide, benzil nicotinate, $\gamma$-orizanol, allantoin, glycyrrhizic acid (salt), glycyrrhetinic acid and its derivative, saponins such as hinokitiol, bi sabot roll, eucalyptol, thymol, inositol, saikosaponin, carrot saponin, sponge gourd saponin, soapberry saponin, pantothenyl ethyl ether, ethynylestradiol, tranexamic acid, arbutin, cepharanthine, and placenta extract; extracts from plants such as Rumex japonicus, Sophora angustifolia, Nuphar japonicum, orange, sage, Achillea sibirica, Malva sylvestris, Swertia japonica, thyme, Ligusticum acutilobum, Picea jezoensis, Birch, Horsetail, a sponge gourd, a horse chestnut, creeping saxifrage, arnica, lily, mugwort, Paeonia lactiflora, aloe, gardenia, and Scomberomorus niphonius; pigments; nonionic surfactants such as sorbitan monolaurate, sorbitan monopalmitate, sorbitan sesquioleate, sorbitan trioleate, polyoxyethylene sorbitan monolaurate, polyoxyethylene sorbitan monostearate, polyethylene glycol monooleate, polyoxyethylene alkylether, polyglycol diether, lauroyl diethanol amide, aliphatic acid isopropanol amide, maltitol hydroxy fatty acid ether, alkylated polysaccharide, alkyl glucoside, and sugar esters; cationic surfactants such as stearyl trimethyl ammonium chloride, benzalkonium chloride, and lauryl amine oxide; anionic surfactants such as sodium palmitate, sodium laurate, sodium laurate, potassium laurylsulfate, triethanolamine alkylsulfate ether, Turkey red oil, linear dodecylbenzene sulfuric acid, polyoxyethylene hydrogenated castor oil maleic acid, and acylmethyl taurine; ampholytic surfactants; perfumes; pigments; and purified water.

Examples of the water-in-oil emulsified composition of the present invention include, but are not limited to, makeup cosmetics, hair cosmetics, skincare cosmetics, and sun care cosmetics.

EXAMPLES

The present invention is described in detail below by referring to Examples, but the present invention is not limited to these Examples. The blend ratios are in wt% units.

Before Examples, testing methods and evaluation criteria used in Examples and Comparative examples are described.

<Sensory test for usability>

A panel of 30 men and 30 women (a total of 60) used the samples obtained from Examples and Comparative examples and evaluated them for smoothness, succulent sensation, and sticky sensation based on the following criteria.

[Evaluation criteria for skin smoothness]

◎ : 30 or more panelists felt skin smoothness. ○ : 10-29 panelists felt skin smoothness. Δ : 5-9 panelists felt skin smoothness. × : Four or less panelists felt skin smoothness.

[Evaluation criteria for succulent sensation of the skin]

◎ : 30 or more panelists felt succulent skin. ○ : 10-29 panelists felt succulent skin. Δ : 5-9 panelists felt succulent skin.× : Four or less panelists felt succulent skin.

[Evaluation criteria for skin stickiness]

◎ : 30 or more panelists felt the absence of skin stickiness. ○ : 10-29 panelists felt the absence of skin stickiness. × : Four or less panelists felt the absence of skin stickiness.

<Emulsification properties>

Emulsification properties of the samples obtained in Examples and Comparative examples were evaluated based on the following evaluation criteria. ○ : Emulsification into the water-in-oil type (W/O type) was homogeneous.

Δ : Emulsification into the water-in-oil type (W/O type) resulted in some water separation and/or oil separation (defective emulsification) × : Emulsification into the water-in-oil type (W/O type) failed.

<Stability>

The samples obtained in Examples and Comparative examples were stored for 30 days under different temperature conditions, i. e. 0˚C, room temperature (25˚C), 37˚C, and 50˚C, and their stability was evaluated based on the following evaluation criteria. ○ : No separation was observed. Δ : Separation was observed. × : Severe separation was observed.

(Preparation of the water phase parts)

Water phase parts 1-3: Water phase parts 1-3 as shown in the following Table 1 were mixed, heated up to 90˚C and dissolved, and then left to cool (slowly cooled) to form a gel. This gel was crushed with a homogenizer to obtain a microgel (average particle size 100 micrometers).

Water phase parts 4 and 5: Water phase parts 4 and 5 as shown in the following Table 1 were mixed and allowed to stand for 12 hours at ordinary temperatures to prepare the water phase parts. Carboxyvinyl polymer used in water phase part 4 and xanthan gum used in water phase part 5 are both thickening compounds that don't have a gelation ability.

Table 1

| | Water phase part 1 | Water phase part 2 | Water phase part 3 | Water phase part 4 | Water phase part 5 |
|---|---|---|---|---|---|
| Agar | 2. 0 | 0. 5 | 0.05 | - | - |
| Carboxyvinyl polymer | - | - | - | 0.6 | - |
| Xanthan gum | - | - | - | - | 1.0 |
| Glycerin | 5. 0 | 5. 0 | 5. 0 | 5.0 | 5. 0 |
| Dipropylene glycol | 5.0 | 5.0 | 5. 0 | - | - |
| Dibutylene glycol | - | - | - | 5. 0 | 5. 0 |
| Methylparaben | Appropriate amount | Appropriate amount | Appropriate amount | Appropriate amount | Appropriate amount |
| TEA | - | - | - | Appropriate amount | Appropriate amount |
| Ion-exchange water | Balance | Balance | Balance | Balance | Balance |
| Preparation method for the water phase parts | Crush (Microgel) | Crush (Microgel) | Crush (Microgel) | Mix and allow to stand | Mix and allow to stand |
| Viscosity (mPa·s/25˚C) | 80,000 | 2,500 | 500 | 80,000 | 2,000 |

(Examples 1-4)

As shown in the following Table 2, (2) - (5) were mixed to obtain the oil phase parts, to which (1) was added and uniformly dispersed, to which the water phase part (6) or (7) was gradually added, followed by uniform emulsification/dispersion with a disper mixer to obtain a water-in-oil emulsified composition. For the "organophilic clay mineral" in Table 2, "Benton 38" (made in Nationalred Co.)was used.

Samples obtained in Examples 1-4 were evaluated for usability (smoothness, moistening sensation, and sticky

sensation), emulsification properties, and stability according to the aforementioned test methods and evaluation criteria. The results are shown in Table 2.

Table 2

| | | Example 1 | Example 2 | Example 3 | Example 4 |
|---|---|---|---|---|---|
| (1) | Organophilic clay mineral (*) | 2. 0 | 2. 0 | 2. 0 | 2. 0 |
| (2) | Dimethicone polyol | 3. 0 | 3. 0 | 3. 0 | 3. 0 |
| (3) | Decamethyl cyclopentasiloxane | 20. 0 | 1 0. 0 | 20. 0 | 10. 0 |
| (4) | Dimethylpolysiloxane (viscosity 6mPa·s/25˚C) | 5. 0 | - | 5. 0 | 5. 0 |
| (5) | Cetyl octanoate | 5. 0 | - | 5. 0 | 5. 0 |
| (6) | Water phase part 1 | 65. 0 | 85.0 | - | - |
| (7) | Water phase part 2 | - | - | 65.0 | 85. 0 |
| Sensation during use | | | | | |
| | Smoothness | ◎ | ◎ | ◎ | ◎ |
| | Moistening sensation | ◎ | ◎ | ○ | ◎ |
| | Sticky sensation | ◎ | ◎ | ◎ | ◎ |
| Emulsification properties | | | | | |
| | Directly after preparation | ○ | ○ | ○ | ○ |
| Stability (30 day storage) | | | | | |
| | 0 ˚C | ○ | ○ | ○ | ○ |
| | Room temperature | ○ | ○ | ○ | ○ |
| | 37˚C | ○ | ○ | ○ | ○ |
| | 50˚C | ○ | ○ | ○ | ○ |

(Comparative examples 1-4 and Example 5)

As shown in the following Table 3, (2)-(5) were mixed to obtain the oil phase parts, to which (1) was added and uniformly dispersed, to which the water phase part selected from (6)-(10) was gradually added, followed by uniform emulsification/dispersion with a disper mixer to obtain a water-in-oil emulsified composition. For the "organophilic clay mineral" in Table 3, "Benton 38" (made in Nationalred Co.) was used.

Samples obtained in Comparative examples 1-4 and Example 5 were evaluated for usability (smoothness, moistening sensation, and sticky sensation), emulsification properties, and stability according to the aforementioned test methods and evaluation criteria. The results are shown in Table 3.

Table 3

| | | Comparative example 1 | Comparative example 2 | Example 5 | Comparative example 3 | Comparative example 4 |
|---|---|---|---|---|---|---|
| (1) | Organophilic clay mineral (*) | 2. 0 | 2. 0 | 2. 0 | 2. 0 | 2.0 |
| (2) | Dimethicone polyol | 1. 0 | 1. 0 | 3. 0 | 3. 0 | 3.0 |
| (3) | Decamethyl cyclopentasiloxane | 5. 0 | 5.0 | 20. 0 | 20. 0 | 20. 0 |
| (4) | Dimethylpolysiloxane (v i scos i ty 6mPa·s/ 25˚C) | - | - | 5. 0 | 5. 0 | 5. 0 |
| (5) | Cetyl octanoate | - | - | 5. 0 | 5. 0 | 5. 0 |
| (6) | Water phase part 1 | 92. 0 | - | - | - | - |
| (7) | Water phase part 2 | - | 92.0 | - | - | - |

(continued)

| | Comparative example 1 | Comparative example 2 | Example 5 | Comparative example 3 | Comparative example 4 |
|---|---|---|---|---|---|
| (8) Water phase part 3 | - | - | 65. 0 | - | - |
| (9) Water phase part 4 | - | - | - | 65.0 | - |
| (10) Water phase part 5 | - | - | - | - | 65.0 |
| Sensation during use | | | | | |
| Smoothness | ○ | ○ | △ | ○ | ○ |
| Moistening sensation | ○ | ○ | △ | △ | △ |
| Sticky sensation | △ | △ | ○ | × | × |
| Emulsification properties | | | | | |
| Directly after preparation | △ | △ | ○ | △ | ○ |
| Stability (30 day storage) | | | | | |
| 0˚C | △ | △ | ○ | △ | △ |
| Room temperature | △ | △ | ○ | △ | △ |
| 37˚C | △ | × | ○ | × | △ |
| 50˚C | × | × | ○ | × | △ |

(Example 6: Moisturizing cream)

| (Ingredients) | | (wt%) |
|---|---|---|
| (1) | Organophilic clay mineral ("Benton-38" from Nationalred Co.) | 2.0 |
| (2) | Diglycerin diisostearic acid | 1.0 |
| (3) | Decamethyl cyclopentasiloxane | 10.0 |
| (4) | Petrolatum | 5.0 |
| (5) | Squalane | 15.0 |
| (6) | Agar | 1.0 |
| (7) | Dipropylene glycol | 5.0 |
| (8) | Methylparaben | 0.2 |
| (9) | Arginine | 0. 1 |
| (10) | Magnesium ascorbate phosphate | 2.0 |
| (11) | Vitamin A | 0. 1 |
| (12) | Perfume | Appropriate amount |
| (13) | Ion-exchange water | Balance |

(Preparation method)

(4), (5), (11), and (12) were mixed and heated up to 50˚C and dissolved, to which (1) was added and uniformly dispersed (oil phase part). Separately, (6)-(10) were added to (13), heated up to 90˚C and dissolved, cooled down to solidify, and crushed with a disper to obtain a microgel having an average particle size of 100 micrometers water phase part). This water phase part (microgel) was gradually added to said oil phase part and dispersed with a disper mixer to obtain a moisturizing cream.

(Example 7: Sunscreen)

| (Ingredients) | | (wt%) |
|---|---|---|
| (1) | Organophilic clay mineral ("Benton 38" from Nationalred Co.) | 2.0 |
| (2) | Dimethicone polyol | 2.0 |
| (3) | Decamethylpentasiloxane | 10.0 |
| (4) | Trimethylsiloxy silicic acid | 5.0 |
| (5) | Hydrophobed titanium oxide | 5.0 |
| (6) | Octyl-p-methoxy cinnamate | 10.0 |
| (7) | Agar | 1.5 |
| (8) | L-arginine hydrochloride | 1.0 |
| (9) | 1,3-butylene glycol | 5.0 |
| (10) | Methylparaben | 0.2 |
| (11) | Evening primrose oil | 0.3 |
| (12) | Spherical polyethylene | 1. 0 |
| (13) | Ascorbic acid glycoside | 2.0 |
| (14) | Tocopheryl acetate | 0.3 |
| (15) | Vitamin A acetate | 0. 1 |
| (16) | Perfume | Appropriate amount |
| (17) | Ion-exchange water | Balance |

(Preparation method)

(1)-(6), (11), (12), (14)-(16) were uniformly dispersed to prepare the oil phase part. Separately, (7)-(10) and (13) were added to (17), heated up to 90˚C and dissolved, cooled down to solidify, and crushed with a disper to obtain a microgel having an average particle size of 60 micrometers (water phase part). This water phase part (microgel) was gradually added to said oil phase part and dispersed with a disper mixer to obtain a sunscreen.

INDUSTRIAL APPLICABILITY

As described in detail above, the present invention can provide a water-in-oil emulsified composition that exhibits good emulsified states, does not change over different temperatures and/or time, has superior stability, and gives a non-sticky, fresh, and good tactile sensation during use, as well as an emulsified cosmetic using this composition.

## Claims

1. A water-in-oil emulsified composition comprising (a) 0. 1-20 wt% organophilic clay mineral, (b) 10-70 wt% oil component, (c) 0.01-10 wt% emulsifier having an HLB value of not more than 7, and (d) 0.1-90 wt% microgel having an average particle size of 0. 1-1, 000 micrometers obtained by dissolving a hydrophilic compound having a gelation ability in water or an aqueous component, letting it cool down and solidify to form a gel, and pulverizing said gel.

2. The water-in-oil emulsified composition of claim 1 wherein the average particle size of the microgel of (d) ingredient is 1-300 micrometers.

3. The water-in-oil emulsified composition of claim 1 or 2 wherein, in (d) ingredient, the hydrophilic compound having gelation ability is one, two, or more selected from a group consisting of agar, carrageenan, curdlan, gelatin, gellan gum, and alginic acid.

4. An emulsified cosmetic comprising the water-in-oil emulsified composition of claim 1, 2, or 3.

Europäisches Patentamt
European Patent Office
Office européen des brevets

## EUROPEAN SEARCH REPORT

Application Number
EP 09 00 0612

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP 2000 219609 A (SHISEIDO CO LTD) 8 August 2000 (2000-08-08) * abstract * | 1-4 | INV. A61Q19/00 A61K8/04 A61K8/06 A61K8/26 A61K8/41 A61K8/65 A61K8/73 |
| Y | JP 11 005712 A (SHISEIDO CO LTD) 12 January 1999 (1999-01-12) * abstract * | 1-4 | |
| Y | JP 10 226622 A (SHISEIDO CO LTD) 25 August 1998 (1998-08-25) * abstract * | 1-4 | |
| Y | EP 1 158 021 A (SHISEIDO CO LTD [JP]) 28 November 2001 (2001-11-28) * paragraphs [0004], [0005], [0018], [0019] * * paragraphs [0021], [0036], [0048] * * claims * | 1-4 | |

TECHNICAL FIELDS SEARCHED (IPC)
A61K
A61Q

The present search report has been drawn up for all claims

1

| Place of search | Date of completion of the search | Examiner |
|---|---|---|
| Munich | 21 April 2009 | Diebold, Alain |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...............................................................
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 09 00 0612

This annex lists the patent family members relating to the patent documents cited in the above-mentioned European search report. The members are as contained in the European Patent Office EDP file on
The European Patent Office is in no way liable for these particulars which are merely given for the purpose of information.

21-04-2009

| Patent document cited in search report | Publication date | Patent family member(s) | Publication date |
|---|---|---|---|
| JP 2000219609 A | 08-08-2000 | NONE | |
| JP 11005712 A | 12-01-1999 | JP 3524717 B2 | 10-05-2004 |
| JP 10226622 A | 25-08-1998 | NONE | |
| EP 1158021 A | 28-11-2001 | WO 0151558 A1 | 19-07-2001 |
| | | TW 279416 B | 21-04-2007 |
| | | US 2009047312 A1 | 19-02-2009 |
| | | US 2003072805 A1 | 17-04-2003 |

EPO FORM P0459

For more details about this annex : see Official Journal of the European Patent Office, No. 12/82